# EUROPEAN PATENT APPLICATION

(11) **EP 1 884 231 A1**
(43) Date of publication of application: **06.02.2008**
(21) Application number: 06118270.5
(22) Date of filing: 01.08.2006
(51) Int. Cl.: A61K 8/97, A61Q 19/08, A61K 8/73

(54) **Cosmetic or pharmaceutical composition containing hyaluronic acid**

(71) Applicant: Auriga International S.A., 1410 Waterloo (BE)
(72) Inventor: Dubois, Jacques, 1495 Villers-la Ville (BE); Marchal, Alfred, 1410 Waterloo (BE)
(74) Representative: Van Malderen, Joëlle

(57) **Abstract**

The present invention is related to a colloidal cosmetic and/or pharmaceutical composition comprising particles of hyaluronic acid combined with a surfactant and its preferred use as skin wrinkles filler or lips filler.

## Description

### Field of the invention

The present invention is related to a (preferably colloidal) cosmetic or pharmaceutical composition, comprising hyaluronic acid particles, preferably made of hyaluronic molecules of at least two different molecular weight combined with a surfactant, preferably a polycation.

The present invention is also related to this composition for the treatment of wound healing and as skin wrinkles or lips filler.

### Background of the invention and state of the art

The hyaluronic acid (also referred as HA or HLA) is a naturally occurring polysaccharide that consists of Na acetyl d-glucosamin and β-glucocoronic acid.

The unique visco-elastic nature of hyaluronic acid along with its biocompatibility and non-immunogenicity has led to its use in a number of clinical applications which include :
- supplementation of joined fluids in arthritis,
- surgical aid in eye surgery and,
- healing and regeneration of surgical wounds.

Applications of hyaluronic acid containing cosmetic are proposed to moisturize and restore elasticity thereby achieving an anti-wrinkle effect. Hyaluronic acid base composition formulation or sunscreen is also used to protect the skin against UVA radiations due to the free radicals binding properties of the hyaluronic acid.

It has also been reported that injection of hyaluronic acid (either in a stabilised form or in combination with other polymers) into the dermis, can reduce facial line and wrinkles in the long term with fewer side effects and better tolerability compared with the use of collagen.

However, this injection could present some side effects, such as allergic reactions possibly due to impurities present in the extracted hyaluronic acid.

Furthermore, it has been also used as a drug delivery agent through various routes of administration (including ophthalmic, nasal, pulmonary, parenteral and topical).

Topical delivery for the treatment of skin disorders offers numerous potential advantages over system therapy, such as those involving the use of oral or parenteral products.

However, the targeted delivery of drugs for the treatment of topical disorders is not trivial.

The physiological function of the stratum corneum, the outer most and none-viable layers of the skin, acts as protective barriers for the body and as such, it is particularly effective at preventing the permeation of hydrophilic molecules, including such drugs into deeper skin layers where viable cells are located.

Therefore, additional methods based upon chemical or physical penetration enhancement are often required to traverse this barrier.

Consequently, in topical applied formulation, there is a need for a system that helps the drugs to negotiate successively the stratum corneum, then avoid further penetration all the way across the deep layer of the skin (the dermis) and into the blood strain.

Obviously, this requires a final balance system that is not easily achieved.

### Aims of the invention

The present invention aims to provide a new hyaluronic cosmetic composition and pharmaceutical composition which does not present the drawbacks of the state of the art and presents improved characteristics, in particular in topical administration, or could be used as a carrier of various active cosmetic and/or therapeutical compounds.

Another aim of the present invention is to provide such cosmetic or pharmaceutical composition comprising hyaluronic acid combined with improved topical administration characteristics.

A further aim of the present invention is to obtain a cosmetic or pharmaceutical composition that improves the visco-elastic properties of human skin (improves moisture and elasticity of the skin) and could be used as an anti-wrinkles filler or as lips filler.

A further aim of the present invention is to provide such composition for the treatment of wound healing.

A further aim of the present invention is to provide such composition having reduced or no side effects and having improved tolerability for human skin.

A last aim of the present invention is to provide such composition which is capable of protecting human skin against ultraviolet (UVA) radiations, but also protects human skin against deleterious effects of free-radicals.

### Summary of the invention

The present invention is related to a (preferably colloidal) cosmetic or pharmaceutical composition comprising particles of hyaluronic acid combined with a surfactant (molecule).

In the composition according to the invention, the hyaluronic particles are molecules having at least two different molecular weight. Preferably, the composition according to the invention is a polydispersed composition made of hyaluronic acid molecules having increased molecular weight (molecular weight gradient), preferably comprised between about 400 Da (one monomeric unit of hyaluronic acid has a molecular weight of 401,329 Da) and about 2 500 000 Da, preferably between about 1 000 Da and about 2 000 000 Da, more preferably between about 10 000 Da and about 1 000 000 Da. More specifically between about 50 000 Da and 1 000 000 Da, more preferably between about 250 000 Da and about 1 000 000 Da.
The inventors have observed unexpectedly that such composition comprising molecules of hyaluronic acid having different molecular weight could be advantageously used (as such) as a cosmetic or pharmaceutical composition, because hyaluronic acid molecules of a specific molecular weight present penetration profile and cosmetic or therapeutical properties defined by their molecular size.

In particular, the inventors have observed that hyaluronic acid molecules having a high molecular weight could be used as a carrier for hyaluronic acid molecules of lower molecular weight, especially hyaluronic acid molecules of low molecular weight associated with a surfactant (molecule),but also as a moisture compound for an efficient hydration of the human skin. Similarly, hyaluronic acid molecules having low molecular weight could be used as carrier for hyaluronic acid molecules of high molecular weight, especially these molecules associated with a surfactant. Usually, molecules which are used as carrier are molecules which are present in excess of molecules associated with the surfactant.

Advantageously, the cosmetic or pharmaceutical composition according to the invention consists only of these particles of hyaluronic acid having preferably different molecular weight, combined with a surfactant (molecule) and possibly with a solvent such as water. A preferred example of this composition contains sodium hyaluronate (10 000 Da) at 0,1%, sodium hyaluronate (10⁶ Da) at 2%, benzyl dimethylhexadecylammonium chloride (BDHDAC) at 1% and water; the percentage being defined according to the weight of the total weight of the composition.

This composition does not need additional cosmetic or pharmaceutical excipients usually included in the composition of the state of the art.

Advantageously, the composition according to the invention does not present any side effects, such as allergy and is made of constituents which are considered as safe and secure for topical administration to mammals, especially to humans.

Preferably, the combination of a surfactant with hyaluronic acid allows the association (complexation) of particles being micels that result into a colloidal composition.

The composition could be a colloidal solution comprising particles having a size comprised between 10⁻⁶ m and 10⁻⁹ m.

A colloid is a fine state of subdivision of particles that are too small to be visible by an ordinary optical microscope and do not settle or settle slowly.

A solution is a solid liquid or gas mixed homogenously with a liquid being a solvent.

A solvent is a substance capable of using dissolving or dispersing one or more substances, especially a liquid component or a solution present in a great amount under the solute.

The colloidal composition according to the invention could be selected from the group consisting of a water in oil emulsion/oil in water emulsion, microemulsion, a clear solution, a suspension of nanoparticles or an anhydrous composition.

A suspension is made of particles mixed in a fluid or in a solid, but undissolved. An emulsion is an ultimate mixture of two incompletely miscible liquids, so one liquid is dispersed with or without an emulsion into the other liquid.

In the composition according to the invention, the surfactant (molecule) is present in a concentration sufficient to allow the formation of micels with hyaluronic acid. The surfactant (molecule) could be any molecule which allows the formation of these micels (such as an amphoteric surfactant or a polycations).

Preferably, the polycation used in the composition according to the invention is selected from the group consisting of quaternary ammonium polycations, polyamines and bases xanthines.

Preferably, the polycation is a quaternary ammonium polycation selected from the group consisting of alkyl ammonium, polyquaternium, acetyl pyridinium, butyl hydroxyl ammonium, cetyl pyridinium, hexadecyl trimethyl ammonium.

Preferred examples of alkyl ammonium are tetra-ethylammonium, tetra-butylammonium, tetrapentylammonium, tetraoctylammonium and other polycations with a longer chain length and with higher molecular weight.

Preferably, polyamines are alkyl amines derivatives, such as diethylamine, dipropylamine, triethylamine, triethanolamine, tripropylamine, tripentylamine, trihexylamine, trioctylamine, tridodecylamine, or other amines with a longer chain length higher than 12 but preferably lower than 50, more preferably lower than 20.

Other examples of polyamines are endogenous polyamines, such as putrecine, spermine and its precursor spermidine, the polylysine, the polyarginine and the polyhistidine, or other examples of natural or synthetic polyamines.

Preferred examples of xanthanes are caffeine, theophylline, theobromine and mateine which are know alkaloids common used for the effect as mild stimulants and as bronchodilators.

According to another preferred embodiment of the present invention, the surfactant (molecule) is the benzyl dimethylhexadecylammonium chloride (BDHDAC) which presents advantageously antimicrobial characteristics and which improves penetration of hyaluronic acid molecules into a mammal skin, especially into a human skin.

Advantageously, this benzyl dimethylhexadecylammonium chloride is combined with hyaluronic acid molecules having low molecular weight, preferably comprised between about 5000 Da and 500 000 Da, more preferably between 10 000 Da and 250 000 Da.

The cosmetic or pharmaceutical composition according to the invention could be also used as a carrier for other active cosmetic or pharmaceutical compound, especially vitamins (such as vitamin C, vitamin E, vitamins K1 or K2, vitamin K1 oxide or vitamin K2 oxide, etc).

Another aspect of the present invention is related to a method for the preparation of the composition according to the invention which comprises the steps of:
- combining a surfactant (molecule) and hyaluronic acid molecule having a low molecular weight, preferably comprised between about 5000 Da and about 500 000 Da, more preferably between 10 000 Da and 250 000 Da to form a complex (preferably the surfactant is the benzyl dimethylhexadecylammonium chloride and the hyaluronic acid molecule has a molecular weight of about 10 000 Da),
- adding to this association (complex) of hyaluronic acid molecules and the surfactant molecule, one or more additional hyaluronic acid molecules having a molecular weight higher than the associated ( complexed) hyaluronic acid molecules, preferably hyaluronic acid molecules having a molecular weight higher than 10 000 Da, preferably higher than 250 000 Da, preferably about 1 000 000 Da; in order to obtain the composition according to the invention, wherein the hyaluronic acid particles of high molecular weight are advantageously used as carriers for hyaluronic acid molecules of low molecular weight.

Preferably, the hyaluronic acid molecules are present in their salt form, preferably as sodium hyaluronate.

According to an other embodiment of the present invention, the composition may comprise portions of hyaluronic acid molecules having a molecular weight comprised between about 200 Da and about 400 Da, more preferably about 250 to 270 Da.
These portions could be used as precursors for the formation of hyaluronic acid molecules.

Furthermore, the composition formulation according to the invention could also comprise other compounds which are skin beneficial ingredients, preferably selected from the group consisting of skin cleansers, skin and hair conditioning agents, vitamins (vitamin C, vitamin E, vitamin K1 or K2, vitamin K1 oxide or vitamin K2 oxide, etc), hormones, minerals, plant extracts, anti-inflammatory agents, concentrates of plant extracts, emollients, moisturizers, skin protectants, humectants, silicones, skin soothing ingredients, analgesics, skin penetration enhancers, solubilizers, emollients, alkaloids, memory enhancers, anesthetics, colorants, perfumes, preservative, seeds, broken seed nut shells, silica, clays, beads, luffa particles, polyethylene balls, mica, pH adjusters, processing aids, and combinations thereof. The quantities of such ingredients can be safe and effective amounts as needed and not limited to any specific limits.

These various elements could be used in order to modify or improve the characteristics of the composition according to the invention.

The composition may also comprise the various known elements which improve the characteristics of a cosmetic or pharmaceutical composition. These elements are for example antioxidants, collagen and elastin synthetis boosters (such as L-tyrosine), various hydroxy acids (alpha hydroxyl acids, beta hydroxyl acids, polyhydroxy acids, vitamins, hormones, skin whitening agents, UVA/UVB sunscreens, antimicrobial agents, antifungal agents, blood microcirculation improvement agents (vasodilatory or vasoconstrictive), skin protectant drug actives, and combinations thereof. Such elements could also comprise elements which promote collagen and elastin in the skin, such as ascorbic acid, ascorbic acid derivatives, glucosamine ascorbate, arginine ascorbate, lysine or tyrosine ascorbate, gluthathione ascorbate, nicotinamide ascorbate, niacin ascorbate, allantoin ascorbate, creatine ascorbate, creatinine ascorbate, chondroitin ascorbate, chitosan ascorbate, DNA ascorbate, carnosine ascorbate, vitamin E, vitamin E derivatives, tocotrienol, rutin, quercetin, hesperedin, diosmin, mangiferin, mangostin, cyanidin, astaxanthin, lutein, lycopene, resveratrol, tetrahydrocurcumin, rosmarinic acid, hypericin, ellagic acid, chlorogenic acid, oleuropein, alpha-lipoic acid, niacinamide lipoate, gluthathione, andrographolide, carnosine, niacinamide, polyphenols, pycnogenol and combinations thereof.

Advantageously, the composition according to the invention could be selected to provide the treatment of a human skin, especially for an efficient hydattation of the skin, combined with an efficient therapeutically or cosmetic effect, skin wrinkles reduction, skin exfoliating, treatment of acne, treatment of rosacea, age-spots reduction, skin surface whitening, skin surface brightening, stria distensae (stretch marks), reduction treatment of pimples, treatment of skin infections and lesions, including surgery lesions, varicose and spider veins reduction, blood microcirculation improvement, UVA/UVB protection of skin, skin redness reduction benefits for the treatment of microbial or fungal contaminants, cellulite control, skin and body toning benefits or combinations thereof, preferably without inducing side effects such as allergy.

The present invention is related to the composition according to the invention for use as a medicament.

Another aspect of the present invention is related to the use of the composition according to the invention in the manufacture of a medicament for the treatment of wound healing, lesions, especially surgery lesions, skin infections, acne, varicose, spider veins and burns.

The composition according to the invention could be used as skin wrinkles filler or lips filler.

Another aspect of the present invention is related to a method of treating skin wrinkles (to obtain a reduction of skin wrinkles)preferably combined with a moisture effect and the skin which comprises the steps of administrating the composition upon the skin of a patient, in order to obtain efficient skin wrinkles reduction, skin wrinkles filling or lips filling.

## Claims

1. A colloidal cosmetic or pharmaceutical composition comprising particles of hyaluronic acid combined with a surfactant.

2. The composition according to the claim 1, wherein the particles of hyaluronic acid combined with a surfactant are micels.

3. The composition according to the claim 1 or 2, wherein the surfactant is a polycation.

4. The composition according to claim 3, wherein the polycations is selected from the group consisting of a quaternary ammonium polycation, a polyamine or a xanthine.

5. The composition according to any of the preceding claims wherein the surfactant is benzyl dimethylhexadecylammonium chloride (BDHDAC).

6. The composition according to any of the preceding claims which further comprises a solvent, preferably water.

7. The composition according to any of the preceding claims which comprises molecules of hyaluronic acid having different molecular weight.

8. The composition according to claim 7 which is a polydispersed composition of hyaluronic acid molecules having a molecular weight comprised between 400 Da and 2 500 000 Da.

9. The composition according to claim 8 wherein the molecules have a molecular weight comprised between about 5 000 Da and 2 000 000 Da.

10. The composition according to claim 7, 8 or 9 wherein the molecules have a molecular comprised between 10 000 Da and 1 000 000 Da.

11. The composition of claims 1 to 10, which is a skin wrinkles filler or a lips filler.

12. Use of the composition according to any of the preceding claims 1 to 11 for the manufacturer of a medicament in the treatment of wounds healing, burns, skin surgery, skin aging, skin exfoliating, treatment of acne, treatment of rosacea, age-spots reduction, skin surface whitening, skin surface brightening, stria distensae (stretch marks), reduction treatment of pimples, treatment of skin infections and lesions, varicose and spider veins reduction, blood microcirculation improvement, UVA/UVB protection of skin, skin redness reduction benefits for the treatment of microbial or fungal contaminants, cellulite control, skin and body toning benefits or combination thereof.
